# EUROPÄISCHE PATENTSCHRIFT

(11) **EP 0 843 713 B1**
(45) Veröffentlichungstag und Bekanntmachung des Hinweises auf die Patenterteilung: **22.03.2000**
(21) Anmeldenummer: 96927666.6
(22) Anmeldetag: 02.08.1996
(51) Int. Cl.: A61K 7/50

(54) **MILDE HAARSHAMPOOS**
MILD HAIR SHAMPOOS
SHAMPOOINGS DOUX

(30) Priorität: 11.08.1995 DE 19529590
(43) Veröffentlichungstag der Anmeldung: 27.05.1998
(73) Patentinhaber: Cognis Deutschland GmbH, 40589 Düsseldorf (DE)
(72) Erfinder: FABRY, Bernd, D-41352 Korschenbroich (DE)
(86) Internationale Anmeldenummer: EP9603417
(87) Internationale Veröffentlichungsnummer: WO9707189

(56) Entgegenhaltungen:
- WO-A-92/06172
- DE-A- 4 340 015

## Beschreibung

### Gebiet der Erfindung

Die Erfindung betrifft Haarshampoos mit verbesserter hautkosmetischer Verträglichkeit, enthaltend C₁₂-C₁₄-Fettsäure-N-alkylpolyhydroxyalkylamide und ausgewählte Zuckertenside sowie die Verwendung der Tensidmischungen zur Herstellung von Haarshampoos.

### Stand der Technik

Fettsäure-N-alkylpolyhydroxyalkylamide und insbesondere Fettsäure-N-alkylglucamide stellen nichtionische Tenside dar, die wegen ihrer guten Detergenseigenschaften zunehmend an Bedeutung gewinnen. Aus der Europäischen Patentanmeldung **EP 0285768 A1** (Hüls) ist beispielsweise ihr Einsatz als Verdickungsmittel bekannt. In der Französischen Offenlegungsschrift **FR 0580491 A1** (Henkel) werden wäßrige Detergensgemische auf Basis von Sulfaten und/oder Sulfonaten, Niotensiden und gegebenenfalls Seifen beschrieben, die Fettsäure-N-alkylglucamide als Schaumregulatoren enthalten. Mischungen von kurz- und längerkettigen Glucamiden werden in der Deutschen Patentschrift **DE 4400632 C1** (Henkel) beschrieben. In den Deutschen Offenlegungsschriften **DE 4236958 A1** und **DE 4309567 A1** (Henkel) wird ferner über den Einsatz von Glucamiden mit längeren Alkylresten als Pseudoceramide in Hautpflegemitteln sowie über Kombinationen von Glucamiden mit Proteinhydrolysaten und kationischen Tensiden in Haarpflegeprodukten berichtet. Gegenstand der Internationalen Patentanmeldungen **WO 92/06153; 06156; 06157; 06158; 06159** und **06160** (Procter & Gamble) sind Mischungen von Fettsäure-N-alkylglucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/06152; 06154; 06155; 06161; 06162; 06164; 06170; 06171** und **06172** (Procter & Gamble) beschrieben.

Glucamide weisen jedoch in Haarpflegemitteln auch in Abmischung mit den bereits genannten Tensiden Mängel in der Schaumhöhe und Schaumbeständigkeit, insbesondere bei Härtebelastung auf. Im übrigen besteht ein Bedarf an Produkten mit weiter verbesserter hautkosmetische Verträglichkeit.

Die Aufgabe der Erfindung hat demnach darin bestanden, einen Weg zu finden, Performance und Hautverträglichkeit von Fettsäure-N-alkylpolyhydroxyalkylamiden im allgemeinen und Fettsäure-N-alkylglucamiden im besonderen signifikant in Haarshampoos zu verbessern.

### Beschreibung der Erfindung

Gegenstand der Erfindung sind milde Haarshampoos, enthaltend 10 bis 30 Gew.-% - bezogen auf die Mittel -
(a) C₁₂-C₁₄-Fettsäure-N-alkylglucamide und
(b) Zuckertenside, ausgewählt aus der Gruppe, die gebildet wird von
   (b1) Saccharoseestern,
   (b2) Sorbitanestern und/oder
   (b3) Polysorbaten
sowie übliche Hilfs- und Zusatzstoffe, mit der Maßgabe, daß das Gewichtsverhältnis der Komponenten (a) und (b) 50 : 50 bis 80 : 20 beträgt.

Überraschenderweise wurde gefunden, daß Abmischungen von C₁₂-C₁₄- Fettsäure-N-alkylglucamiden und den genannten Zuckertensiden in Haarshampoos zu einer in synergistischer Weise verbesserten hautkosmetischen Verträglichkeit bei gesteigertem Schaumvermögen führt.

### Fettsäure-N-alkylglucamide

Fettsäure-N-alkylglucamide stellen nichtionische Tenside dar, die der Formel **(I)** folgen, 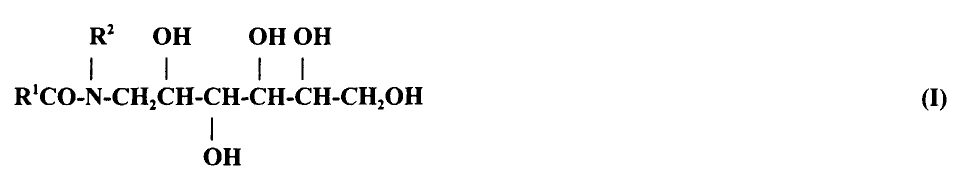 in der R¹CO für einen aliphatischen Acylrest mit 12 bis 14 Kohlenstoffatomen und R² für Wasserstoff, einen Alkyl- oder Hydroxyalkylrest mit 1 bis 4 Kohlenstoffatomen steht.

Bei den Fettsäure-N-alkylglucamiden handelt es sich um bekannte Stoffe, die üblicherweise durch reduktive Aminierung eines reduzierenden Zuckers mit 5 oder 6 Kohlenstoffatomen, insbesondere der Glucose, mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H. Kelkenberg findet sich in **Tens. Surf. Det. 25, 8 (1988)**.

Vorzugsweise werden Fettsäure-N-alkylglucamide eingesetzt, in der R² für Wasserstoff oder eine Alkylgruppe steht und R¹CO für den Acylrest der Laurinsäure oder der Myristinsäure bzw. derer technischer Mischungen steht. Besonders bevorzugt sind Fettsäure-N-alkylglucamide, die durch reduktive Aminierung von Glucose mit Methylamin und anschließende Acylierung mit Laurinsäure oder C_{12/14}-Kokosfettsäure bzw. einem entsprechenden Derivat erhalten werden.

### Saccharoseester

Saccharoseester, vielfach auch als Zuckerester bezeichnet, stellen seit langer Zeit bekannte nichtionische Tenside dar. Üblicherweise erfolgt die Herstellung dieser Verbindungen über den Weg der Umesterung von Fettsäuremethylestern mit Saccharose in Gegenwart von basischen Katalysatoren wie beispielsweise Pyridin oder Piperidin in organischen Lösungsmittel [vgl. **DE 2051766 AS** (Day-Ichi; zu lösungsmittelfreien Methoden vgl. L. Guillardeau in **Tens. Surf. Det. 29, 342 (1992)**]. Je nach Auswahl der Rohstoffe und Reaktionsbedingungen entstehen dabei Mono-, Di-, Tri- und Polyester der Saccharose, wobei in der Regel nur die Mono- und Diester eine anwendungstechnische Bedeutung besitzen. Eine Übersicht zu diesem Thema ist beispielsweise von N. Desai in **Parf. Kosm. 64, 463 (1991)** erschienen, zu Struktur und Eigenschaften der Stoffe vgl. auch **Surfactants in Consumer Products, J.Falbe (ed.), Springer-Verlag, Berlin, 1987, S. 101-103.**
Die Saccharoseester folgen der Formel **(II)**, 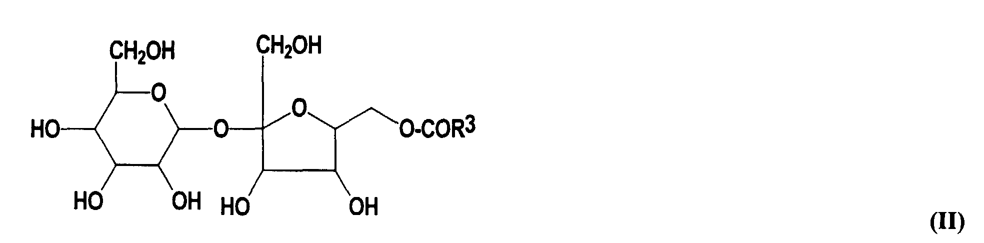 in der R³CO für einen aliphatischen Acylrest mit 6 bis 22 Kohlenstoffatomen und 0 und/oder 1, 2 oder 3 Doppelbindungen steht. Formel **(II)** gibt exemplarisch einen Monoester wieder. Es versteht sich, daß auch die weiteren Hydroxylgruppen, insbesondere die primären Hydroxylgruppen verestert sein können.

Typische Beispiele für geeignete Saccharoseester, vorzugsweise Saccharosemono- und/ oder -diester sind Umesterungsprodukte der Saccharose mit Methylestern der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Bevorzugt sind technische Saccharosemono- und/oder -diester, deren Fettsäurekomponente sich von einem technischen Kokosfettsäureschnitten mit 12 bis 14 bzw. 12 bis 18 Kohlenstoffatomen ableitet.

### Sorbitanester

Sorbitanester stellen Veresterungs- bzw. Umesterungsprodukte von Sorbitol mit Fettsäuren bzw. Fettsäuremethylestern bei Temperaturen von 200 bis 250 °C dar. Dabei wird in einem ersten Schritt Sorbitol zu einer Mischung von 1,4- und 3,6- Sorbitan dehydratisiert [vgl. S. Ropuszynski et al. in **Tens. Surf. Det. 27, 350 (1990)**] und das Sorbitan anschließend - abhängig von den Einsatzbedingungen - in ein Gemisch von Mono-, Di- und Triestern überführt [vgl. C. Akoh et al. in **J. Am. Oil. Chem. Soc., 66, 1581 (1989)** und **Surfactants in Consumer Products, J. Falbe (ed), Springer-Verlag, Berlin, 1987, S. 101-103**.

Die Sorbitanester enthalten einen aliphatischen Acylrest R⁴CO mit 6 bis 22 Kohlenstoffatomen und 0 und/oder 1, 2 oder 3 Doppelbindungen. Es kommen Monoester in Frage, es können jedoch ebenfalls die weiteren Hydroxylgruppen verestert sein.

Typische Beispiele für geeignete Sorbitanester, vorzugsweise Sorbitanmono-, Sorbitansesqui- und/oder Sorbitandiester, sind formale Veresterungsprodukte des Sorbitans mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelenschen Oxosynthese oder der Dimerisierung von ungesättigten Fettsäuren anfallen.

Bevorzugt sind technische Sorbitanmono-, -sesqui- oder -diester, deren Fettsäurekomponenten sich von einem technischen Kokosfettsäureschnittmit 12 bis 14 bzw. 12 bis 18 Kohlenstoffatomen, Laurinsäure, Palmitinsäure, Stearinsäure oder Ölsäure ableiten.

### Polysorbate

Polysorbate stellen ethoxylierte Sorbitanester dar und werden üblicherweise durch nachträgliche Anlagerung von Ethylenoxid an die freien Hydroxylgruppen der Sorbitanester bzw. durch Insertion in die Carbonylesterbindungen hergestellt. Die Polysorbate enthalten einen aliphatischen Acylrest R⁵CO mit 6 bis 22 Kohlenstoffatomen und 0 und/oder 1, 2 oder 3 Doppelbindungen und die Summe der Ethylenoxideinheiten steht für Zahlen von 1 bis 50, vorzugsweise 10 bis 25. Es kommen Monoester in Frage, es können jedoch ebenfalls die weiteren Hydroxylgruppen verestert sein.

Typische Beispiele für geeignete Polysorbate, vorzugsweise ethoxylierte Sorbitanmono-, Sorbitansesqui- und/oder Sorbitandiester, sind Anlagerungsprodukte von durchschnittlich 1 bis 50, vorzugsweise 1 bis 15 Mol Ethylenoxid an jeweils 1 Mol eines formalen Veresterungsproduktes des Sorbitans mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelenschen Oxosynthese oder der Dimerisierung von ungesättigten Fettsäuren anfallen.

Bevorzugt sind technische ethoxylierte Sorbitanmono-, -sesqui- oder -diester, deren Fettsäurekomponente sich von einem technischen Kokosfettsäureschnitt mit 12 bis 14 bzw. 12 bis 18 Kohlenstoffatomen, Laurinsäure, Palmitinsäure, Stearinsäure oder Ölsäure ableitet und die 10 bis 25 Mol Ethylenoxid pro Mol Ester enthalten.

Die erfindungsgemäßen Detergensgemische können die Fettsäure-N-alkylpolyhydroxyalkylamide und die ausgewählten Zuckertenside im Gewichtsverhältnis 50 : 50 bis 80 : 20 enthalten.

### Tenside

Die erfindungsgemäßen Detergensgemische können weitere anionische, nichtionische, kationische und/oder amphotere Tenside enthalten.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Proteinhydrolysate (insbesondere pflanzliche Produkte auf Weizenbasis), Polyolfettsäureester und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J. Falbe (ed.), "Surfactants in Consumer Products", Springer Verlag**, **Berlin, 1987, S. 54-124** oder J. Falbe (ed.), "**Katalysatoren, Tenside und Mineralöladditive", Thieme Verlag, Stuttgart, 1978, S. 123-217** verwiesen.

Die erfindungsgemäßen Mittel können die oben genannten zusätzlichen Tenside in Anteilen von 1 bis 50, vorzugsweise 5 bis 25 Gew.-% - bezogen auf den Feststoffanteil der Gemische - enthalten.

### Gewerbliche Anwendbarkeit

Die erfindungsgemäßen Haarshampoos zeichnen sich durch ein besonders vorteilhaftes Schaumvermögen und eine in synergistischer Weise verbesserte hautkosmetische Verträglichkeit aus. Sie enthalten 10 bis 30 Gew.-% - bezogen auf die Mittel - C₁₂-C₁₄-Fettsäure-N-alkylglucamide und Zuckertenside, wobei das Gewichtsverhältnis 50 : 50 bis 80 : 20 beträgt, sowie die folgenden Hilfs- und Zusatzstoffe:

Haarshampoos können als weitere Hilfs- und Zusatzstoffe - neben den bereits genannten Tensiden - Emulgatoren, Überfettungsmittel, Verdickungsmittel, Kationpolymere, Siliconverbindungen, biogene Wirkstoffe, Filmbildner, Konservierungsmittel, Farb- und Duftstoffe enthalten.

Als **Emulgatoren** kommen sowohl bekannte W/O- als auch O/W- Emulgatoren wie beispielsweise gehärtetes und ethoxyliertes Ricinusöl, Polyglycerinfettsäureester oder Polyglycerinpolyricinoleate bzw. Polyglycerinpoly-12-hydroxystearate in Frage. Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Geeignete **Verdikkungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid. Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US. Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, yaluronsäure bzw. deren Salze und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

Ein letzter Gegenstand der Erfindung betrifft schließlich die Verwendung der Tensidmischungen zur Herstellung von milden Haarschampoos, enthaltend 10 bis 30 Gew.-% - bezogen auf die Mittel -
(a) C₁₂-C₁₄-Fettsäure-N-alkylglucamide und
(b) Zuckertenside, ausgewählt aus der Gruppe, die gebildet wird von
   (b1) Saccharoseestern,
   (b2) Sorbitanestern und/oder
   (b3) Polysorbaten
sowie übliche Hilfs- und Zusatzstoffe, mit der Maßgabe, daß das Gewichtsverhältnis der Komponenten (a) und (b) 50 : 50 bis 80 : 20 beträgt.

### Beispiele

### I. Eingesetzte Tenside

A1) Laurinsäure-N-methylglucamid
B1) Saccharose-mono/di-laurat
B2) Sorbitanmonolaurat DEHYMULS® SML, Henkel KGaA, Düsseldorf/FRG
B3) Polysorbat (Sorbitanmonolaurat-20 EO) EUMULGIN® SML-20. Henkel KGaA, Düsseldorf/FRG

### II. Anwendungstechnische Ergebnisse

Das Schaumvermögen wurde nach der DIN-Methode 53 902, Teil 2 (Ross-Miles-Test) durchgeführt. Eingesetzt wurden 1 Gew.-%ige Tensidlösungen in Wasser von 16°d; die Temperatur betrug 20° C. Bestimmt wurden Basisschaum und Schaumvolumen nach 5 min.

Die Bestimmung des Reizpotentials erfolgte gemäß der OECD-Methode No.404 und der EEC Directive 84/449 EEC, Pt.B.4. Die angegebenen Reizsummenscores wurden aus den nach 24, 48 und 72 Stunden erhaltenen Reizscores gebildet. Dabei wurde der im Vergleichsversuch V1 ermittelte Reizsummenscore für ein 100 %iges C_{12/18}-Kokosfettsäuremonoglyceridsulfat-Natriumsalz zu 100 % gesetzt und die in den übrigen Versuchen erhaltenen Reizsummenscores zu diesem ins Verhältnis gesetzt.

Die Ergebnisse sind in Tabelle 1 zusammengefaßt (Prozentangaben als Gew.-%).

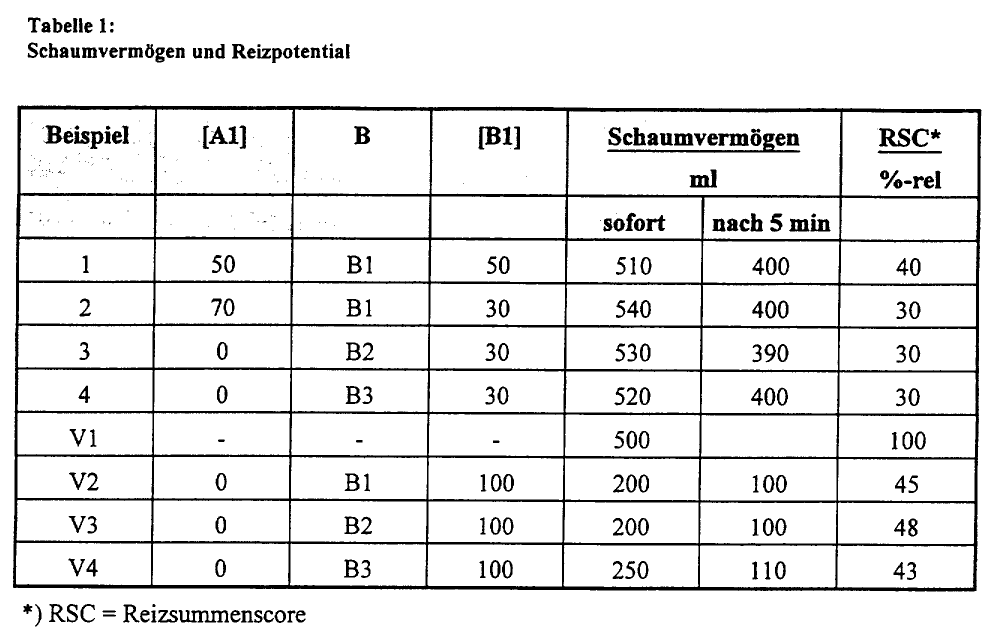

## Patentansprüche

1. Milde Haarshampoos, enthaltend 10 bis 30 Gew.-% - bezogen auf die Mittel -
(a) C₁₂-C₁₄-Fettsäure-N-alkylglucamide und
(b) Zuckertenside, ausgewählt aus der Gruppe, die gebildet wird von
(b1) Saccharoseestern,
(b2) Sorbitanestern und/oder
(b3) Polysorbaten
sowie übliche Hilfs- und Zusatzstoffe, mit der Maßgabe, daß das Gewichtsverhältnis der Komponenten (a) und (b) 50 : 50 bis 80 : 20 beträgt.

2. Haarshampoos nach Anspruch 1, **dadurch gekennzeichnet**, daß sie Saccharoseester der Formel **(II)** enthalten, 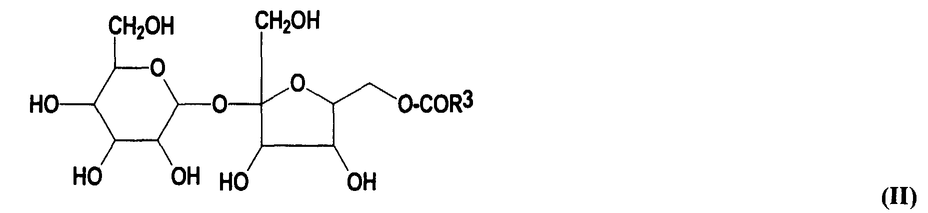 in der R³CO für einen aliphatischen Acylrest mit 6 bis 22 Kohlenstoffatomen und 0 und/oder 1, 2 oder 3 Doppelbindungen steht.

3. Verwendung von Tensidmischungen zur Herstellung von milden Haarshampoos, enthaltend 10 bis 30 Gew.-% - bezogen auf die Mittel -
(a) C₁₂-C₁₄-Fettsäure-N-alkylglucamide und
(b) Zuckertenside, ausgewählt aus der Gruppe, die gebildet wird von
(b1) Saccharoseestern,
(b2) Sorbitanestern und/oder
(b3) Polysorbaten
sowie übliche Hilfs- und Zusatzstoffe, mit der Maßgabe, daß das Gewichtsverhältnis der Komponenten (a) und (b) 50 : 50 bis 80 : 20 beträgt.

## Claims

1. Mild hair shampoos containing 10 to 30% by weight, based on the shampoo, of
(a) C₁₂₋₁₄ fatty acid-N-alkyl glucamides and
(b) sugar surfactants selected from the group consisting of
(b1) sucrose esters,
(b2) sorbitan esters and/or
(b3) polysorbates
and typical auxiliaries and additives, with the proviso that the ratio by weight of component (a) to component (b) is 50:50 to 80:20.

2. Shampoos as claimed in claim 1, characterized in that they contain sucrose esters corresponding to formula (II): 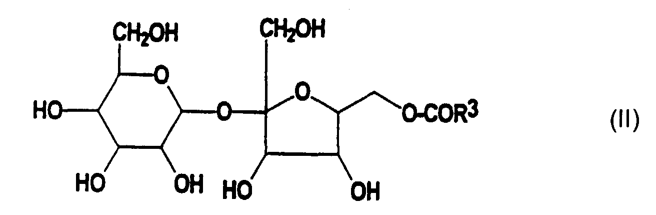 in which R³CO is an aliphatic acyl group containing 6 to 22 carbon atoms and 0 and/or 1,2 or 3 double bonds,

3. The use of surfactant mixtures for the production of mild hair shampoos containing 10 to 30% by weight - based on the shampoo - of
(a) C₁₂₋₁₄ fatty acid-N-alkyl glucamides and
(b) sugar surfactants selected from the group consisting of
(b1) sucrose esters,
(b2) sorbitan esters and/or
(b3) polysorbates
and typical auxiliaries and additives, with the proviso that the ratio by weight of component (a) to component (b) is 50:50 to 80:20.

## Revendications

1. Shampooing doux pour les cheveux contenant de 10 à 30 % en poids rapporté à la composition :
a) de N-alkylglucamide d'acides gras en C₁₂-C₁₄, et
b) des agents tensioactifs dérivés de sucre choisis dans le groupe formé par :
b1) des esters de saccharose
b2) des esters de sorbitanne, et/ou
b3) des polysorbates
ainsi que des adjuvants et des additifs usuels avec la réserve que le rapport en poids des composants a) et b) est de 50 : 50 à 80 : 20.

2. Shampooings pour les cheveux selon la revendication 1,
caractérisé en ce qu'
ils renferment des esters de saccharose de formule (II) : 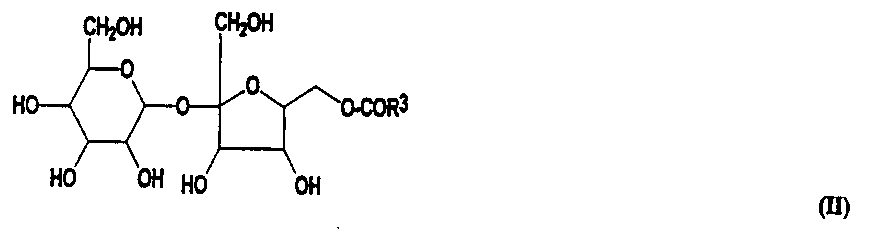 dans laquelle R³CO représente un reste acyle aliphatique ayant de 6 à 22 atomes de carbone et 0 et/ou 1, 2 ou 3 double liaisons.

3. Utilisation de mélanges d'agents tensioactifs pour la préparation de shampooings doux pour cheveux, renfermant de 10 à 30 % en poids, rapporté à la composition de :
a) N-alkylglucamides d'acides gras en C₁₂-C₁₄, et
b) des agents tensioactifs dérivés de sucre choisis dans le groupe formé par :
b1) des esters de saccharose,
b2) des esters de sorbitanne, et/ou
b3) des polysorbates,
ainsi que des adjuvants et des additifs usuels avec la réserve que le rapport en poids des composants a) et b) est de 50 : 50 à 80 : 20.
